Europäisches Patentamt

European Patent Office

Office européen des brevets

(19)

(11) **EP 0 740 959 A1**

(12) **DEMANDE DE BREVET EUROPEEN**

(43) Date de publication:
06.11.1996 Bulletin 1996/45

(51) Int. Cl.$^6$: **B01L 11/00**, G01N 27/28,
B65D 83/08

(21) Numéro de dépôt: 96106413.6

(22) Date de dépôt: 24.04.1996

(84) Etats contractants désignés:
BE CH DE DK ES GB IT LI NL SE

(30) Priorité: 02.05.1995 FR 9505222

(71) Demandeur: ASULAB S.A.
CH-2501 Bienne (CH)

(72) Inventeur: Jaeger,Gérard
1807 Blonay (CH)

(74) Mandataire: Patry, Didier Marcel Pierre et al
I C B,
Ingénieurs Conseils en Brevets S.A.
Rue des Sors 7
2074 Marin (CH)

(54) **Appareil distributeur permettant avec une économie maximale d'utiliser des zones successives d'une bande consommable**

(57) Cet appareil comprend des moyens de commande (58, 74, 75) couplés de préférence à un couvercle (22) pour faire reculer la bande consommable (34) dans l'appareil avant de détacher une zone consommable (Z1 à Z9) de cette bande.

La bande (34) peut être avancée pas-à-pas après son introduction dans l'appareil, par l'intermédiaire d'un mécanisme d'avance (30) pouvant être actionné par un bouton coulissant (23) et agissant sur un curseur (35) auquel la bande (34) peut être accouplée et qui peut circuler dans un couloir (29) ménagé dans le boîtier 20 de l'appareil.

Application notamment à des appareils de distribution de médicaments ou des glucomètres à usage des diabétiques.

FIG.:3

EP 0 740 959 A1

## Description

La présente invention est relative à un appareil distributeur nécessitant pour fonctionner l'emploi d'éléments consommables se présentant sous la forme de bande, chacune de ces bandes comprenant dans le sens longitudinal plusieurs zones d'utilisation successives destinées à être séparées de la bande dès après leur utilisation.

Plus particulièrement, l'invention concerne un tel appareil formant un dispositif de mesure permettant de mesurer un paramètre d'une substance déposée sur des zones successives d'une bande formant capteur de mesure consommable, appelé aussi capteur multizone.

Un tel dispositif de mesure peut avantageusement servir à la mesure du taux de glucose dans le sang, à l'usage des diabétiques. Il a été décrit sous différents aspects dans plusieurs demandes de brevet français déposées au nom de la Demanderesse et parmi lesquelles l'on peut citer le FR 92 01 331 pour ce qui concerne le procédé de mesure électrochimique utilisé, le FR 93 11 316 pour ce qui concerne un dispositif de coupe permettant de séparer de la bande consommable les zones de mesure utilisées, le FR 93 11 317 pour ce qui concerne un dispositif de connexion électrique permettant de relier la bande captrice à un circuit électronique de mesure destiné à élaborer le résultat de la mesure sous une forme perceptible à un utilisateur, et enfin le FR 93 11 319 qui concerne plus spécifiquement un dispositif d'éjection du dernier tronçon de la bande captrice, lorsque toutes les zones de mesure ont été utilisées.

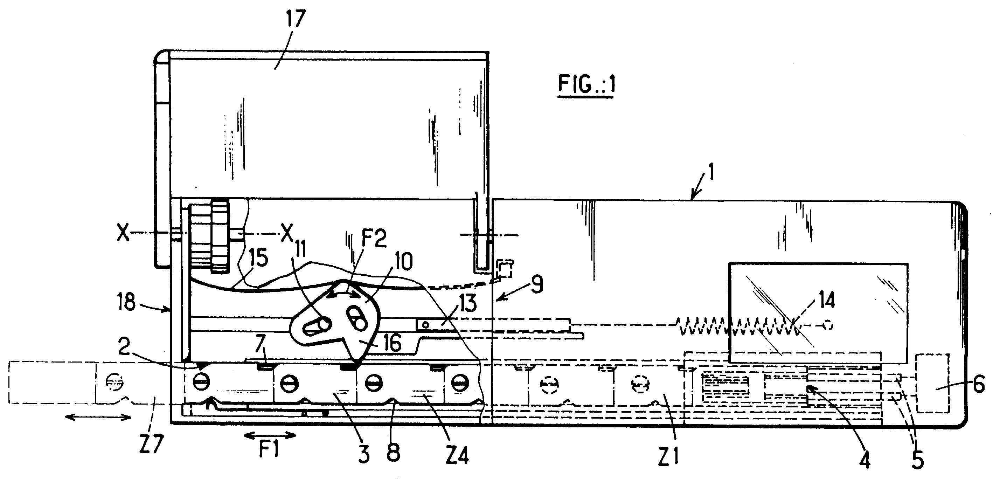

La figure 1 des dessins annexés montre un dispositif de mesure dans lequel on retrouve, sous une forme sommaire, un exemple de réalisation des perfectionnements qui ont fait l'objet des demandes de brevet susindiquées, étant entendu que pour en trouver une description détaillée, l'on peut se référer aux textes desdites demandes de brevet.

Ainsi, le dispositif de mesure comporte un boîtier 1, de forme générale allongée et d'une taille telle qu'il puisse facilement être tenu dans le creux de la main d'un adulte. Ce boîtier définit un couloir 2 de circulation orienté longitudinalement dans le boîtier 1 et destiné à la circulation d'une bande captrice 3 (double flèche F1) ainsi que d'un curseur 4. Ce dernier est chargé de transmettre le signal électrique provenant de la bande captrice 3 à des lignes électriques 5 s'étendant le long du couloir 2. Ces lignes sont à leur tour connectées à un circuit électronique 6 destiné à exploiter convenablement ce signal électrique pour le rendre intelligible à un utilisateur au moyen d'un dispositif d'affichage prévu également dans le boîtier 1.

Dans l'exemple de la figure 1, la bande captrice 3 est conformée spécifiquement à la mesure du taux de glucose dans le sang. Une description détaillée d'une telle bande peut être trouvée dans la première demande de brevet précitée. Il suffit de retenir ici qu'elle comporte plusieurs zones de mesure, sept en l'occurrence, référencées Z1 à Z7 dont la première Z7 représentée en pointillés sur la figure 1 est supposée avoir déjà été utilisée et donc tronçonnée de la bande 3.

On remarquera également que la bande captrice comprend pour toutes les zones Z1 à Z7 un cran d'avancement 7 (figure 1A) sur l'un de ses bords longitudinaux ainsi que pour toutes les zones, une encoche de positionnement 8 située sur l'autre bord de la bande.

Les crans d'avancement 7 coopèrent avec un mécanisme 9 d'avance longitudinal de la bande 3 le long de son couloir 2. Ce dernier comprend un bouton d'actionnement (non visible sur la figure 1) monté en coulissement longitudinal dans le boîtier, c'est-à-dire comme vu sur la figure 1, au dessus du couloir 2.

Le bouton d'actionnement coopère avec un cliquet basculant 10 guidé sur deux tétons 11 et 12 solidaires d'une réglette 13. Celle-ci est couplée au bouton d'actionnement et montée coulissante dans le boîtier 1 dans la direction de la flèche F1 à l'encontre de l'action d'un ressort de rappel 14 ancré sur le boîtier 1. Le cliquet basculant 10 est maintenu en position non active (représentée sur la figure 1) par un ressort à lame 15 et comporte un bec d'actionnement 16 destiné à coopérer avec les crans d'avancement 7 de la bande captrice 3.

Dès lors, on comprend que le bouton d'actionnement étant activé en va et vient, la bande captrice avance de la longueur d'une zone de mesure de la bande, le mécanisme d'avance faisant basculer le cliquet 10 en va et vient (selon la flèche F2) pour d'abord pousser la bande hors du couloir d'un pas, puis revenir vers la position inactive telle que représentée en se dégageant de la bande.

Le dispositif comprend aussi un couvercle 17 monté rotatif sur le boîtier autour d'un axe X-X et couplé mécaniquement à un mécanisme de coupe 18 permettant de tronçonner la zone de la bande consommable que l'on vient d'utiliser, et ce par un simple mouvement de fermeture du couvercle 17.

Il est encore à noter que le curseur 4 coulisse librement dans le couloir 2 et est couplé mécaniquement à la bande captrice consommable 3 lors de son insertion dans l'appareil, lorsque le curseur 4 vient buter contre le fond du couloir 2. A cet effet, le curseur porte un organe de couplage élastique cédant sous la force d'insertion exercée sur la bande 3 par l'utilisateur, pour s'accrocher à cette bande dès que la force d'insertion est relâchée.

La figure 1A des dessins annexés montre une bande captrice 3 de la technique antérieure avant qu'elle ne soit insérée dans l'appareil.

Chaque zone présente une longueur r choisie pour que lorsque la zone est sortie de l'appareil pour être utilisée, l'utilisateur dispose de suffisamment de place pour effectuer l'action qu'il doit accomplir sur cette zone. Dans l'application spécifique décrite ici, cette action est le dépôt d'une goutte de sang dans la région m où la mesure du taux de glucose doit être effectuée. La longueur r doit être égale au pas d'avance de la bande, lorsqu'elle est déplacée pour présenter la zone de mesure suivante.

On notera cependant que la région m dans laquelle se déroule réellement la mesure est loin d'occuper l'entière surface de la zone et que pour la commodité de l'opération, cette région se trouvera de préférence le plus près possible de l'extrémité de la zone opposée à celle par laquelle elle s'introduit dans le couloir 2.

En d'autres termes, cette région n'occupe qu'une longueur s de la bande, la région vide dans chaque zone de la bande ayant une longueur t qui n'a une utilité que pour respecter la distance sur laquelle la zone doit sortir de l'appareil au cours de son utilisation ( r=s+t ). Il en résulte que la bande doit avoir une longueur totale nettement plus grande que la longueur totale strictement nécessaire pour les sept régions de mesure m de la bande.

Cette exigence se répercute directement sur la longueur que doit présenter le couloir 2 et par voie de conséquence sur la longueur totale de l'appareil. Or, il est clair que pour l'utilisateur un appareil avec des dimensions aussi petites que possible est un avantage, en particulier dans le contexte de l'application décrite ici, car dans ce cas, l'appareil se trouvera le plus souvent dans la poche ou dans le sac à main de l'utilisateur/trice.

L'invention a donc pour but de fournir un appareil distributeur utilisant des bandes consommables de faible longueur pour un nombre de zones de mesure donné, et présentant de ce fait également une faible longueur.

L'invention a donc pour objet un appareil distributeur nécessitant pour fonctionner l'emploi d'éléments consommables se présentant sous forme de bandes, chacune de ces bandes comprenant dans le sens longitudinal plusieurs zones d'utilisation successives destinées à être séparées de la bande dès après leur utilisation, ledit appareil comprenant :

- un boîtier définissant un couloir de circulation pour lesdites bandes, ledit couloir présentant, compte tenu du sens d'introduction desdites bandes, une extrémité amont et une extrémité aval,
- un mécanisme d'avance pour faire sortir ladite bande du couloir de circulation selon un mouvement pas-à-pas après son introduction dans celui-ci par un utilisateur, et
- une unité coulissante montée mobile dans ledit couloir, couplée fonctionnellement audit mécanisme d'avance et destinée à être accouplée à l'extrémité aval de ladite bande lors de son introduction par l'utilisateur,
- ledit appareil distributeur étant caractérisé en ce qu'il comporte également des moyens de commande pour permettre le recul de ladite bande sur une distance prédéterminée inférieure à un pas d'avancement de cette bande après qu'elle ait exécutée un tel pas.

Grâce à ces caractéristiques, chaque zone de la bande peut occuper une longueur comparativement plus faible que par le passé. En effet, pendant qu'une zone n de la bande est utilisée, celle-ci peut être sortie de l'appareil sur une longueur supérieure à la longueur de la zone concernée, pendant que la zone n+1 est également sortie, au moins sur une certaine partie de sa longueur. Après utilisation de la zone n, la zone n+1 peut alors être ramenée à l'intérieur de l'appareil par les moyens de recul, ce qui la protège contre toute détérioration.

Dans ces conditions, il devient possible de prévoir sur la bande plus de zones qu'il n'était possible de le faire par le passé. Dans un exemple pratique, où la bande comporte neuf zones, on peut ainsi la raccourcir par exemple d'environ 50 mm ou alternativement pour une même longueur de la bande, y prévoir davantage de zones. Si l'on choisit la première solution, une bande plus courte permet de réduire la taille de l'appareil, le rendant ainsi d'une utilisation plus commode.

D'autres caractéristiques et avantages de l'invention apparaîtront au cours de la description qui va suivre, donnée uniquement à titre d'exemple et faite en se référant aux dessins annexés sur lesquels :

- la figure 1, déjà décrite, est une vue schématique en plan, avec arrachement partiel, d'un appareil distributeur conforme aux demandes de brevet précitées;
- la figure 1A est une vue en plan montrant une bande consommable selon la technique antérieure, destinée à être utilisée dans l'appareil distributeur de la figure 1;
- les figures 2A, 2B et 2C sont trois vues extérieures, selon trois plans orthogonaux, d'un appareil distributeur selon l'invention;
- la figure 3 est une vue en plan d'un appareil distributeur selon l'invention, la partie supérieure de son boîtier étant supposée enlevée et certaines parties en étant représentées avec arrachement partiel;
- la figure 4 est une vue en plan, à échelle agrandie, de l'appareil selon l'invention, et notamment de la construction du mécanisme d'avance;
- les figure 5 et 5A sont des vues en coupe prises respectivement selon les lignes V-V et VA-VA de la figure 4;
- les figures 6 et 6A sont des vues partielles respectivement en perspective et en vue extérieure de l'appareil, montrant plus particulièrement le couvercle et le mécanisme de coupe;
- la figure 7 illustre plus particulièrement les avantages que l'invention permet d'obtenir;
- la figure 8 montre en quatre vues, la cinématique des moyens de déplacement de la bande consommable, utilisés dans l'appareil suivant l'invention.
- les figures 9A et 9B montrent par des vues orthogonales respectives une variante de l'invention;
- les figures 10A et 10B montrent des vues analogues d'une autre variante; et
- la figure 11 montre une troisième variante de l'invention.

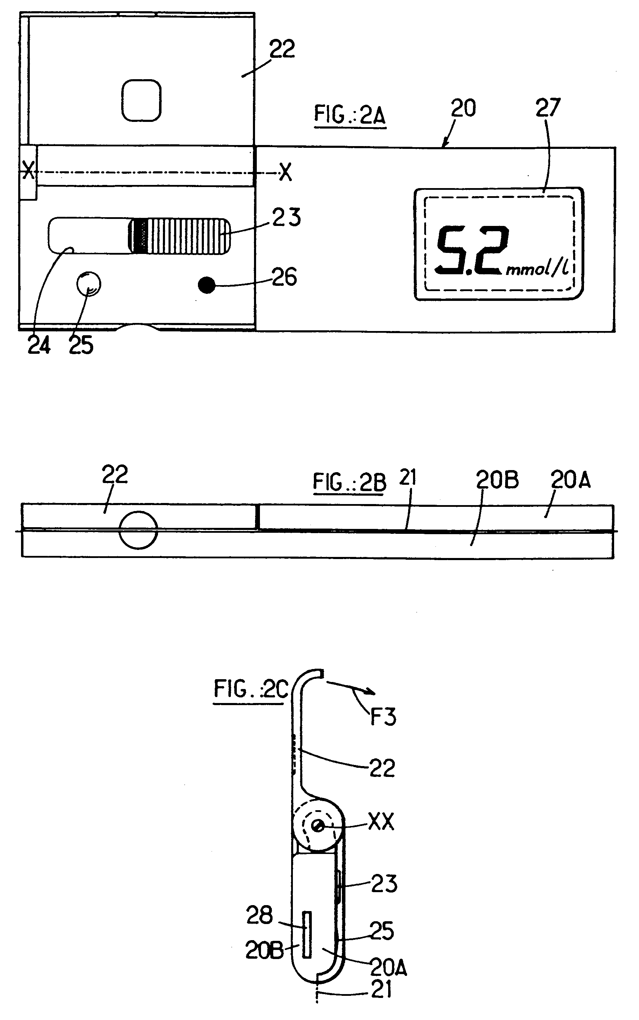

Les figures 2 à 6 représentent un exemple préféré de réalisation de l'appareil distributeur suivant l'invention dans son application à un dispositif permettant de mesurer le taux de glucose dans le sang à l'usage des diabétiques, par exemple. Cependant, l'invention n'est pas limitée à cette application spécifique. En effet, elle peut être mise en oeuvre, dans toutes sortes d'autres cas dans lesquels il est souhaitable d'utiliser des bandes consommables sur lesquelles des zones adjacentes définissent une aire d'utilisation spécifique, ces zones, après avoir été utilisées, pouvant, voire devant (pour des raisons médicales par exemple) être ôtées de la bande pour laisser la place à une zone suivante. Par exemple, dans le domaine médicale, un tel appareil pourrait être utilisé pour la distribution d'une série de cachets ou pilules de médicaments devant, selon la posologie prescrite par le médecin, être pris dans un ordre strict défini à l'avance. Toutefois, l'invention ne se limite pas davantage aux seules applications dans le domaine médical.

L'appareil distributeur selon l'invention représenté aux figures 2 à 6 comporte un boîtier 20 fait en deux demi-coquilles 20A et 20B, en matière plastique moulée de préférence, et assemblées l'une à l'autre selon un plan de joint 21. Dans l'application décrite, le boîtier 20 a une forme allongée et présente des dimensions telles qu'il puisse facilement être tenu dans le creux de la main d'un adulte.

L'appareil comporte un couvercle 22 s'étendant sur une partie de sa longueur et articulé sur le boîtier 20 autour d'un axe longitudinal X-X.

Sur les figures 2A et 2C, on a représenté le couvercle 22 en position ouverte et il peut être fermé en le faisant tourner dans le sens de la flèche F3. Lorsqu'il est ouvert, on aperçoit un bouton 23 coulissant dans un guide 24 ménagé dans la demi-coquille supérieure 20A du boîtier 20. Une petite loupe 25 permet d'observer une partie de la bande consommable (non représentée ici) glissée dans l'appareil. Un poussoir 26 est destiné à rappeler des mesures effectuées antérieurement et stockées dans la mémoire de l'appareil, pour l'affichage sur un écran 27 servant à permettre également la lecture immédiate des résultats de la mesure qui vient d'être faite.

Il est à noter que l'appareil est mis en état de marche, dès que le bouton 23 est actionné et que son alimentation (par une pile incorporée, de préférence) est coupée après l'écoulement d'un délai prédéterminé (60 secondes par exemple) défini à partir du moment où toute intervention sur l'appareil par l'utilisateur a cessé.

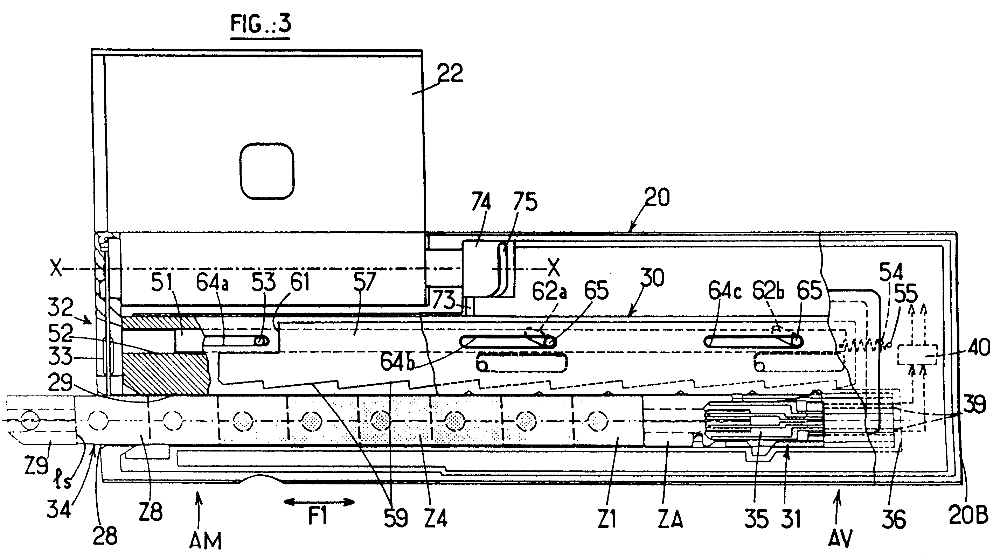

Du côté du couvercle 22, sur le chant frontal latéral du boîtier 20, l'appareil présente une ouverture 28 qui est l'embouchure d'un couloir de circulation 29 (figure 3) des bandes consommables.

La figure 3 montre l'appareil distributeur selon l'invention, alors que l'on en a ôté la demi-coquille supérieure 20A. De la sorte, on peut observer plusieurs unités fonctionnelles et notamment:

- un mécanisme de déplacement 30 qui permet d'avancer pas-à-pas une bande consommable dans le couloir 29 sous l'action répétée du bouton coulissant 23, et également d'assurer le déplacement en sens inverse;

- une unité coulissante 31, destinée à être accouplée à une bande consommable et coulissant dans le couloir 29;

- une unité de coupe 32 située le long du chant d'extrémité de l'appareil et comprenant un couteau 33 monté coulissant dans une direction perpendiculaire à l'axe X-X; et

- une bande consommable 34 présentant des zones de mesure Z1, Z2....etc. et une zone ZA formant amorce et permettant son couplage à l'unité coulissante 31.

Celle-ci 31 comprend un curseur 35 pouvant circuler dans le couloir 29 qui est obturé à son extrémité 36 opposée à l'embouchure 28. Dans ce qui va suivre, on appellera cette extrémité, "extrémité aval" AV en considérant le mouvement d'introduction de la bande consommable 34 dans le couloir 29. De même, l'extrémité opposée, près de l'embouchure 28 sera appelée "extrémité amont" AM. Ces termes seront également utilisés pour la bande consommable, son extrémité aval étant donc située près de la zone d'amorce ZA.

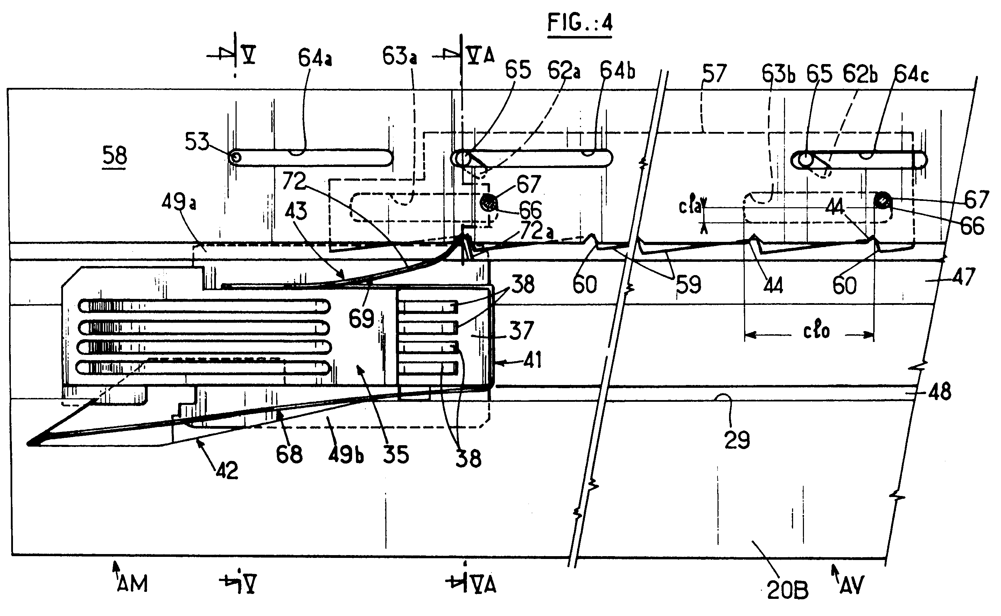

Le curseur 35 comprend un corps de curseur 37 (figure 4) portant des moyens électriques de transmission 38 chargés d'assurer le prélèvement des signaux électriques de la bande 34 et la transmission de ces signaux à des pistes de contact 39 qui courent le long de la paroi supérieure du couloir 29. Ces pistes 39 sont connectées à un circuit électronique de traitement 40 (figure 3) relié à son tour à l'écran d'affichage 27. Selon une variante, on peut également prévoir un câble souple pour assurer la transmission des signaux, ce câble se déployant dans le couloir 29 au fur et à mesure que le curseur atteint l'extrémité amont AM de ce couloir.

Le corps 37 porte également des moyens élastiques 41 de blocage et de couplage qui coopèrent avec des moyens 42 formant butée venus de moulage dans la demi-coquille inférieure 20B du boîtier 20.

Le corps 37 porte en outre des moyens 43 d'entraînement et de positionnement destinés à coopérer avec les moyens de déplacement 30 et avec des crans d'indexage 44 prévus le long du couloir 29 et définissant respectivement les positions de mesure de la bande 34.

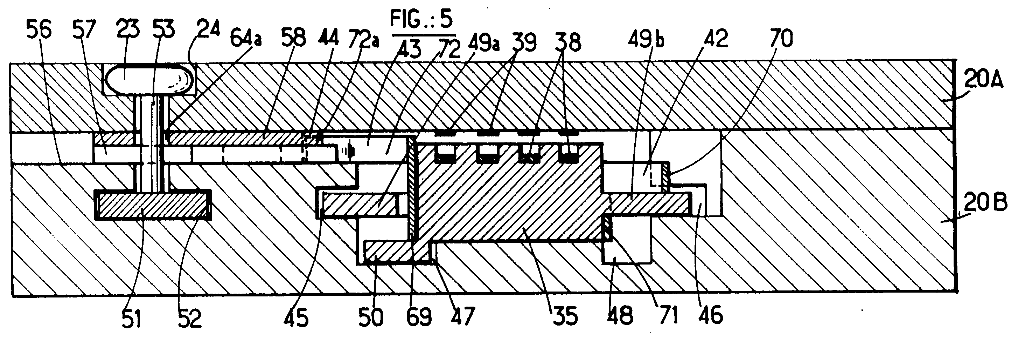

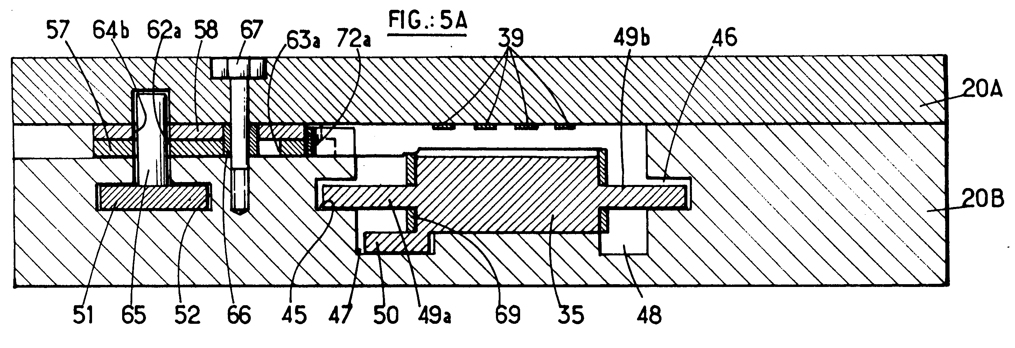

Sur les figures 4, 5 et 5A en particulier, on voit que le couloir de guidage 29 présente deux rainures longitudinales latérales 45, 46 et deux rainures longitudinales 47 et 48 ménagées dans son fond. Le corps 37 est de forme générale parallélépipédique d'où font saillie deux nervures latérales 49a et 49b, s'ajustant respectivement dans les rainures latérales 45 et 46 et une nervure inférieure 50 qui s'ajuste dans la rainure 47 prévue dans le fond du couloir 29.

Les moyens de déplacement 30 comprennent une réglette 51 de section rectangulaire montée mobile dans une rainure longitudinale 52 à section en forme de T inversé et prévue dans la demi-coquille 20B. Cette réglette 51 est solidaire d'une première tige 53 faisant saillie hors de la rainure 52 et passant à travers la demi-coquille 20A pour être engagée à force dans le bouton coulissant 23.

La réglette 51 est sollicitée en permanence dans le sens AV sous l'action d'un ressort de rappel 54 logé, côté aval, dans la rainure 52 (figure 3). Ce ressort 54 est accroché à la réglette 51 par une extrémité, tandis que son extrémité opposée est fixée sur un petit ergot 55 venu de moulage avec la demi-coquille 20B et situé à l'extrémité aval de la rainure 52.

Une partie de la surface supérieure de la demi-coquille 20B forme une surface de glissement 56 s'étendant le long de la rainure 29. Elle sert d'appui à une barre d'encliquetage 57 destinée à coulisser en va et vient selon la direction de la double flèche F1.

Cette barre d'encliquetage 57 est prise entre la surface de glissement 56 et une plaque de guidage allongée 58 disposée au-dessus de la barre d'encliquetage 57. Cette plaque de guidage 58 est également montée coulissante dans le boîtier de l'appareil dans la direction de la flèche F1, tandis que les crans de positionnement 44 pour l'unité coulissante 31 sont prévus dans son bord.

Le bord de la barre d'encliquetage 57 longeant la rainure 29 présente une denture 59 à dents de scie, le flanc court 60 de chaque dent étant situé du côté aval AV.

Le nombre des dents de cette denture est égal au nombre de zones prévues sur une bande consommable 34 ayant la longueur maximale acceptable par l'appareil. Il est à noter au passage que celui-ci peut accepter des bandes consommables de toutes les longueurs entre cette longueur maximale et celle correspondant à un nombre minimale de zones qu'il est raisonnablement utile de prévoir sur la bande consommable (En théorie, on pourrait ne prévoir qu'une seule zone sur la bande).

La barre d'encliquetage présente également:

- une découpe avant 61 (figure 3) s'étendant dans le sens de la longueur de la barre, au dessus de l'ouverture de la rainure en forme de T 52 pour notamment permettre le mouvement de la tige 53, lorsque le bouton 23 est actionné;
- deux lumières 62a et 62b en forme de boutonnière placées obliquement par rapport à la direction longitudinale générale de la barre, c'est-à-dire à la direction de la double flèche F1; et
- deux découpes 63a et 63b de forme rectangulaire servant à limiter la course longitudinale clo et la course latérale cla de la barre d'encliquetage 57.

La plaque 58 présente trois lumières allongées 64a, 64b et 64c, alignées les unes sur les autres dans le sens longitudinal, donc selon la direction de la double flèche F1.

La première de ces lumières 64a est traversée par la tige 53 déjà mentionnée. La seconde 64b et la troisième 64c sont traversées par des tiges d'entraînement 65 passant respectivement également dans les boutonnières 62a et 62b de la barre d'encliquetage 57. Elles sont fixées sur la réglette 51 et se déplacent donc de concert avec le bouton 23.

Les découpes 63a et 63b sont traversées par des douilles 66 qui servent d'entretoises et à travers lesquelles passent également des vis de serrage 67 vissées dans la demi-coquille 20B. Ces douilles permettent à la barre 57 et à la plaque 58 de se déplacer dans leur propre plan sans trop de frottements.

Comme déjà indiqué ci-dessus, la longueur des découpes 63a et 63b définit la course longitudinale clo de la barre d'encliquetage 57, tandis que leur largeur en détermine la course latérale cla (figure 4). De cette manière, les découpes 63a et 63b en coopération avec les douilles 66, ainsi que les deux boutonnières 62a et 62b assurent la définition du mouvement de la barre 57 selon un trajet orbital de tracé rectangulaire.

Les moyens élastiques 41 de blocage et de couplage et les moyens 43 d'entraînement et de positionnement sont de préférence réalisés dans une même lame élastique. Celle-ci présente une forme en U et peut par exemple être surmoulée par de la matière plastique au moment du formage du curseur 35. L'âme du U est appliquée contre la face aval du curseur 35 tandis que ses branches 68 et 69 s'étendent vers l'embouchure 28 du couloir 29 en longeant respectivement les flancs latéraux du curseur 35.

La branche 68 comporte deux ressorts 70 et 71 destinés à assurer le blocage du curseur 35 dans la position amont AM et à le libérer dès l'insertion d'une bande consommable dans le couloir 29. Cet aspect de l'appareil ne faisant pas partie de l'invention, on ne le décrira pas en détail ici.

La branche 69 constitue en fait les moyens 43 d'entraînement et de positionnement déjà mentionnés. Elle comprend un ressort 72 s'étendant vers l'aval et se terminant par un crochet 72a. Celui-ci est destiné à coopérer avec les crans 44 de la plaque 58 et également avec la denture 59 de la barre d'encliquetage 57.

On notera que les rives de la bande consommable ne comportent aucune encoche ou autre élément nécessaire pour l'entraînement de la bande. En effet, cet entraînement est assuré exclusivement par l'intermédiaire du curseur 35 sur lequel agit le mécanisme de déplacement 31. La forme de la bande consommable est donc des plus simples.

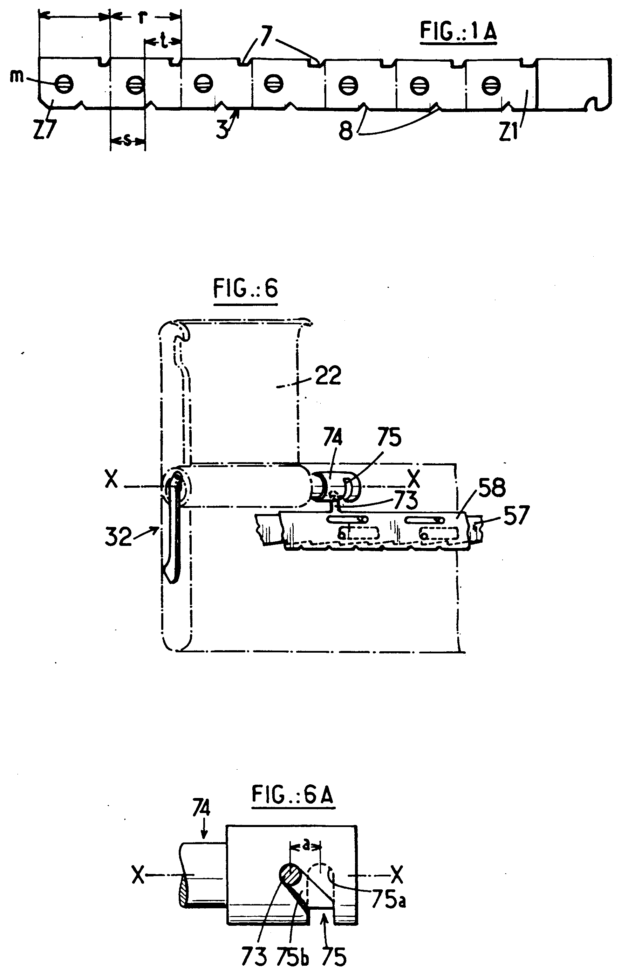

La figure 6 en particulier montre que, selon une caractéristique particulière de l'invention, la plaque de guidage 58 comprend un doigt latéral 73 qui s'étend à partir de l'un de ses bords latéraux en direction de l'axe X-X d'articulation du couvercle 22.

Du côté opposé à l'unité de coupe 32, ce dernier comporte un prolongement cylindrique 74 dont la surface cylindrique, d'axe X-X également, comporte une rainure 75 dont une première partie 75a est profilée dans un plan radial par rapport à l'axe X-X et dont une seconde partie 75b présente un profil hélicoïdal d'axe X-X. Le doigt 73 présente une longueur telle que son extrémité pénètre dans la rainure 75 qui lui sert ainsi de came.

Angulairement, la rainure est placée de telle manière que, lorsque le couvercle 22 est fermé, le doigt 74 se trouve à l'extrémité de la partie droite 75a éloignée de la partie hélicoïdale 75b.

Par contre, lorsque le couvercle 22 est en position ouverte, le doigt 73 se trouve à l'extrémité de la partie hélicoïdale 75b opposée à la partie droite 75a.

Ceci a pour effet que lors du mouvement d'ouverture du couvercle 22, le doigt 74, et par conséquent la plaque de guidage 58, sont déplacés du côté aval AV vers le côté amont AM et inversement, lors du mouvement de fermeture, la plaque 58 se déplace en sens inverse c'est-à-dire qu'elle recule en considérant le mouvement de sortie de la bande consommable 34.

Les deux mouvements provoquent un déplacement, dans un sens ou dans l'autre, des encoches 44 le long du couloir 29, ce dont il résulte en définitive un déplacement dans le sens correspondant du curseur 35 le long du couloir 29. En effet, par son crochet 72a, le ressort 72 est toujours en prise avec l'une de ces encoches 44, tant que le bouton 23 n'est pas actionné.

Il est à noter toutefois que pour coordonner le mouvement longitudinal du curseur 35 avec l'opération de coupe effectuée par l'unité de coupe 32, les mouvements ne se produisent que pendant une partie du mouvement de rotation du couvercle 22, à savoir pendant que le doigt 73 se trouve dans la partie hélicoïdale 75b de la rainure 75. C'est pourquoi, l'emplacement angulaire de cette partie hélicoïdale 75b de la rainure 75 doit être choisi avec soin.

L'amplitude du mouvement longitudinal est choisie notamment en fonction de la distance souhaitable sur laquelle une zone donnée de la bande consommable doit sortir de l'appareil pour que l'utilisateur puisse commodément effectuer l'opération correspondante. Par exemple dans le cadre de l'exemple spécifique de la mesure du taux de glucose, l'utilisateur doit facilement pouvoir déposer une goutte de sang sur la région de mesure m de chaque zone.

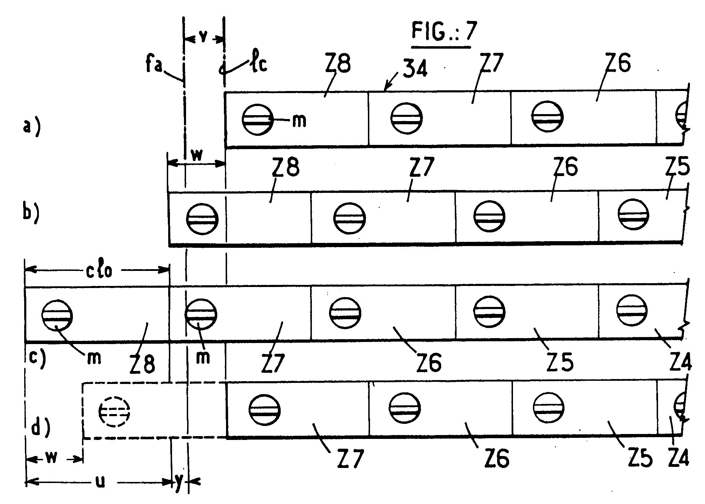

En se référant à la figure 7, on admet que cette distance est égale à u par exemple, compté à partir de la face amont fa du boîtier 20. Cette face fa se situe à une certaine distance v de la ligne de coupe 1c du couteau de l'unité de coupe 32, compte tenu notamment de l'épaisseur de la paroi du boîtier 20 à cette endroit.

Sur la figure 7, on a représenté en a) la position de la bande consommable 34 quand vient d'en être tronçonnée la zone Z9. Le bord amont de cette zone (toujours considéré dans le sens de l'introduction de la bande 34) se trouve alors aligné sur la ligne de coupe 1c. Le couvercle 22 est encore fermé.

L'utilisateur ouvre alors l'appareil en faisant basculer le couvercle 22. Ceci fait avancer la bande consommable 32 d'une distance w égale à la longueur axiale de la partie hélicoïdale 75b de la rainure 75. Cette situation apparaît en b) sur la figure 7. On voit que la distance w est légèrement supérieure à la distance v d'une quantité y, par exemple.

Pendant cette opération d'ouverture du couvercle 22, le doigt 73 est déplacé entraînant ainsi la plaque de guidage 58 et par l'intermédiaire du cran correspondant 44 de celle-ci et du ressort 72, le curseur 35 ainsi que la bande 34.

Ensuite, le bouton 23 est actionné pour faire avancer la bande consommable d'un pas d'une longueur d'une zone (c'est-à-dire la longueur clo, le fonctionnement à cet égard étant décrit en détail ci-après). La bande se trouve alors dans la position représentée sur la figure 7 en c). La longueur de sortie de la bande est alors égale à u=clo+y, c'est-à-dire suffisante pour permettre une utilisation commode de la région de mesure m.

L'utilisateur peut alors effectuer l'opération adéquate sur la bande consommable, par exemple déposer une goutte de sang sur la région de mesure m de la zone Z8, enlever une pilule ou un autre objet analogue fixé sur la bande dans cette zone, etc. Cette opération est facilitée du fait que la région de mesure m de la zone Z8 se trouve relativement éloignée de la face fa du boîtier. On notera que, dans l'exemple représenté, la région de mesure m de la zone suivante Z7 reste en deçà de la face avant fa du boîtier à l'intérieur de celui-ci. La région de mesure de cette zone Z7 ne peut donc pas se détériorer par une mauvaise manipulation.

Après achèvement de l'opération de mesure, l'utilisateur peut alors refermer l'appareil, ce qui provoque d'abord le recul de la bande sur la distance w, puis le tronçonnage de la zone Z8 par l'unité de coupe 32. La zone Z7 est alors prête à être employée (figure 7 en d).

La figure 7 montre clairement l'avantage que procure la disposition selon l'invention. En effet, sans le mouvement d'avance et surtout le mouvement de recul de la bande sur la distance w après l'utilisation de la zone concernée, il faudrait avancer la bande d'un pas égale à w+clo. En d'autres termes selon l'invention chaque zone de la bande peut être plus courte de w.

Un exemple pratique montre que cet avantage est important. En supposant que le déplacement w dû au mouvement du couvercle soit de 7 mm, il est possible de gagner 7 mm par zone. Si on souhaite disposer de bandes ayant neuf zones, par exemple, on peut alors gagner 63 mm en longueur de bande et réduire d'autant la dimension correspondante du boîtier. Alternativement, pour une même longueur de bande, on peut augmenter de façon correspondante le nombre de zones de chaque bande.

Il est à noter que dans le mode de réalisation que l'on vient de décrire, la bande subit nécessairement un mouvement d'avance, puis un mouvement de recul du fait de la rotation du couvercle 22. Cependant, il est possible non seulement de dissocier les deux mouvements l'un de l'autre, mais également de les provoquer séparément par d'autres moyens mécaniques que ceux décrits ci-dessus. Par exemple le mouvement d'avance préalable pourrait être joint à celui effectué à l'aide du bouton 23. Par ailleurs, le mouvement de recul avant la coupe pourrait être réalisé moyennant d'autres moyens mécaniques actionnés d'une autre façon.

On va maintenant décrire plus en détail le fonctionnement du mécanisme d'avance ou de déplacement 30.

Sur la figure 3, on a représenté schématiquement l'appareil, alors que l'utilisateur vient d'y insérer une bande consommable. Toutefois, la bande n'est pas encore tout à fait parvenue au bout du couloir 29. Pour cela, la ligne de séparation ls (fictive) entre les zones Z8 et Z9 doit encore être placée devant le tranchant de l'unité de coupe 32. Le mécanisme d'avance est au repos; la barre d'encliquetage 57 est donc dans sa position aval, sa denture 59 étant effacée hors du couloir 29.

En revanche, sur la figure 4, le curseur 35 se trouve dans la position extrême amont dans le couloir 29 sans qu'une bande ne soit présente dans l'appareil. Cependant, on suppose dans cette figure que l'utilisateur vient de manoeuvrer le bouton 23 pour la dernière fois, que la zone d'amorce ZA a déjà été éjectée de l'appareil, mais que l'utilisateur n'a pas encore lâché le bouton 23. De ce fait, dans cette configuration, la barre d'encliquetage 57 se trouve encore dans sa position amont AM, le ressort de rappel 54 est tendu et le petit flanc 60 de la dent la plus amont de la denture 59 est encore en prise avec le crochet 72a du ressort 72. Par ailleurs, la tige 53 engagée dans le bouton 23 se trouve à l'extrémité amont de la rainure 64a de la plaque de guidage 58.

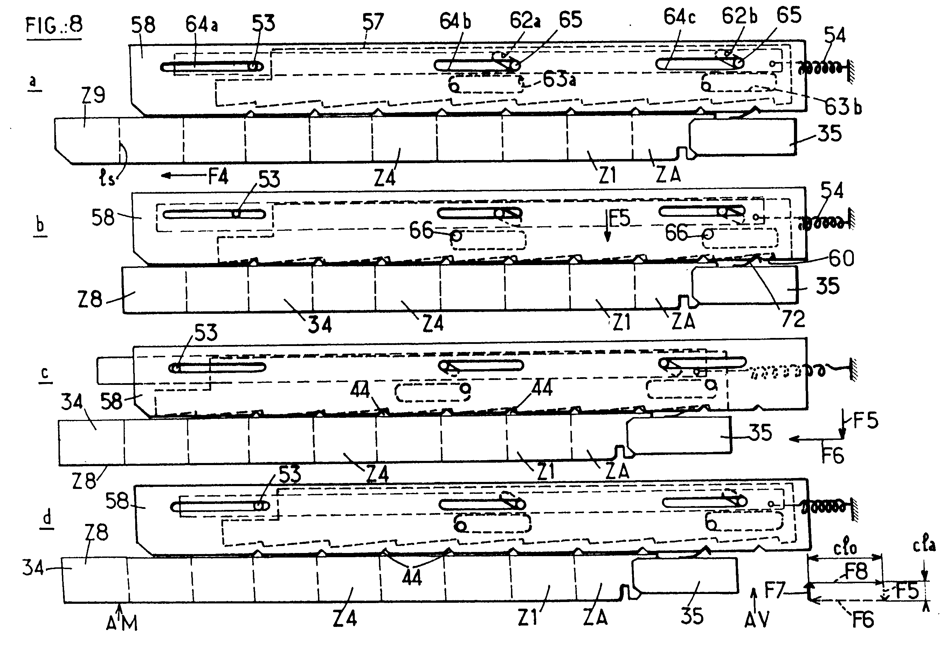

Les quatre vues a à d de la figure 8 représentent les positions principales des composants de l'appareil selon l'invention au cours d'un cycle d'avance de la bande consommable provoqué uniquement par le mouvement du bouton 23. Comme on l'a vu précédemment, à ce cycle vient s'ajouter la course d'avance ou de recul due aux mouvement d'ouverture et de fermeture du couvercle 22, ces mouvement ayant volontairement été omis dans cette figure pour ne pas en compliquer la lecture. Sur les quatre représentations de la figure 8, il est donc supposé que le couvercle est maintenu ouvert.

Plus précisément, sur la figure 8a et par rapport à la figure 3, la bande consommable 34 se trouve complètement insérée dans l'appareil, le curseur 35 étant donc à fond de course à l'extrémité aval du couloir 29.

Le ressort 54 maintient la barre d'encliquetage 57 dans la position aval et en raison de l'obliquité des lumières 62a et 62b, cette barre occupe la position latérale extérieure par rapport au couloir 29.

La zone Z9 de la bande qui fait alors saillie hors de l'appareil peut donc être utilisée pour une mesure du taux de glucose. L'utilisateur ayant terminée cette mesure, il ferme le couvercle 22 ce qui entraîne le processus déjà décrit à propos de la figure 7 (recul de la bande et coupure de la zone Z9 le long de la ligne de séparation ls avec la zone Z8).

L'utilisateur, lors d'une nouvelle utilisation de l'appareil, doit alors en faire sortir la zone Z8. Pour ce faire, il ouvre le couvercle 22 (avance de la bande sur la distance w, non représentée sur la figure 8), puis il actionne le bouton 23 dans le sens de la flèche F4 (figure 8b). Il en résulte d'abord la configuration des composants représentée sur cette figure, configuration qui n'est que fugitive, mais qui montre que la barre d'encliquetage 57 se déplace d'abord latéralement vers l'intérieur dans le sens de la flèche F5 sur la course cla, de nouveau grâce à l'obliquité des lumières 62a et 62b. La denture 59 est alors placée dans la région d'action du ressort 72.

Le bouton 23 continuant à être avancé, le flanc 60 le plus en aval de la denture 59 va ensuite venir en contact du crochet 72a du ressort 72, à l'encontre de la force du ressort de rappel 54 qui est attaché à la réglette 51, puis ce crochet exerce une force d'entraînement sur le curseur 35. Celui-ci se déplace donc vers l'extrémité amont AM du couloir 29 en poussant la bande consommable 34 devant lui (sens de la flèche F6).

Le mouvement dans ce sens s'arrête, lorsque la course clo est accomplie, c'est-à-dire quand les bords aval des découpes 63a et 63b viennent en contact des douilles 66 engagées autour des vis 67. Cette course correspondant exactement à la longueur clo d'une zone sur la bande consommable 34, la zone Z8 se trouvera donc hors de l'appareil.

L'utilisateur n'a plus alors qu'à lâcher le bouton 23 ce qui ramène la barre d'encliquetage 57 vers sa position de départ sous l'action du ressort 54 et selon le trajet symbolisé par les flèches F7 et F8 sur la figure 8d. Par ailleurs, le crochet 72a du ressort 72 dont est équipé le curseur 37 s'étant placé dans le cran d'indexage 44 correspondant à la zone Z8 de la bande consommable, celle-ci reste stable dans sa nouvelle position, tant que le couvercle n'est pas fermé.

On voit donc que ce cycle de fonctionnement implique un mouvement orbital de la barre d'encliquetage selon un tracé qui présente une courbe fermée rectangulaire selon les flèches F5 à F8 représentées sur la figure 6.

Bien entendu, ce cycle peut se répéter pour toutes les zones de mesure de la bande consommable 34, jusqu'à ce qu'il ne reste plus que la zone d'amorce ZA dans l'appareil. Cependant, dans la position correspondante du curseur 35, cette zone est découplée de celui-ci et l'utilisateur peut donc l'ôter facilement de l'appareil pour pouvoir y introduire une nouvelle bande consommable.

Dans le mode de réalisation que l'on vient de décrire, la course sur laquelle la bande consommable peut être déplacée par le mouvement de rotation du couvercle est déterminée par la longueur axiale de la partie hélicoïdale de la rainure 75. Cette course est relativement faible du fait qu'une partie du mouvement de rotation du couvercle est consacrée à l'actionnement du tranchant de l'unité de coupe 32. Or, il est évident que la course de la bande consommable sur la distance w doit être accomplie avant que ce tranchant n'entame la bande. Ceci est possible grâce au fait que l'entraînement du tranchant par le couvercle 22 est du type bielle-manivelle de sorte qu'au début du mouvement de fermeture du couvercle, la course en rotation du couvercle est proportionnellement faible par rapport à la course rectiligne du tranchant. Pendant cette partie du mouvement, le tranchant peut donc se rapprocher de la bande, pendant que celle-ci avance linéairement sur la distance w du fait que le doigt 72 suit la partie hélicoïdale 75b de la rainure 75.

Cependant, il s'avère que l'angle de rotation du couvercle ne permet pas d'obtenir une distance w importante, alors que dans certains cas, une distance w aussi importante que possible serait souhaitable.

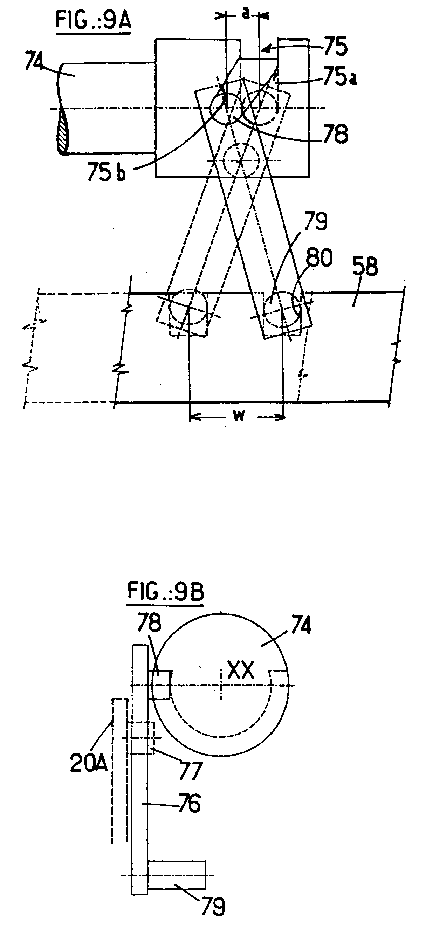

Ce résultat peut être obtenu grâce aux variantes de l'invention représentées sur les figures 9A à 11.

Les figures 9A et 9B montrent une première variante du couplage entre le couvercle 22 et la plaque de guidage 58.

Comme dans le mode de réalisation décrit ci-dessus, le couvercle 22 comporte un prolongement cylindrique 74 munie d'une rainure 75 avec une partie radiale 75a et une partie hélicoïdale 75b. Ce mécanisme comporte un levier articulé 76 monté rotatif sur un pivot 77 venu de moulage dans la demi-coquille supérieure 20A du boîtier 20. Ce levier 76 comporte un premier téton 78 engagé dans la rainure 75 et un deuxième téton 79 engagé dans une encoche 80 prévue dans la plaque de guidage 58. Dans ce cas pour un débattement longitudinal w de la plaque de guidage 58 la longueur axiale a de la rainure 75 peut être plus faible dans le rapport des longueurs correspondantes du levier 76 de part et d'autre de son centre de rotation. Ce levier sert donc d'amplificateur de mouvement.

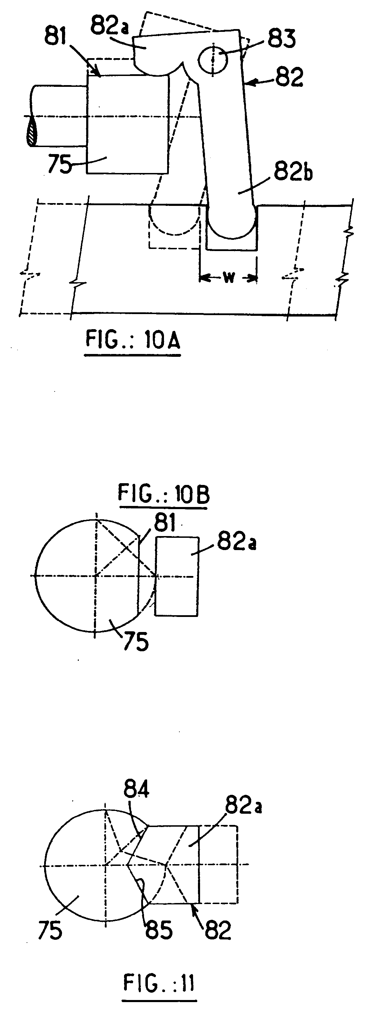

Une variante de ce dispositif d'amplification de mouvement est représentée sur les figures 10A et 10B. Dans ce cas, le prolongement 75 de l'axe du couvercle 22 comporte un méplat 81. Un levier 82 en forme de L est articulé sur un pivot 83 solidaire de la demi-coquille supérieure 20A. Sa petite branche 82a vient appuyer sur la surface périphérique du prolongement 75, tandis sa grande branche 82b est engagée par son extrémité dans l'encoche 80 de la plaque de guidage 58. On comprend que ce dispositif constitue également un amplificateur de mouvement, la distance a' correspondant à l'amplitude de mouvement de la petite branche 82a lors de la rotation du couvercle 22, étant majorée du rapport des longueurs des deux branches du levier 82, pour arriver à un déplacement w de la plaque de guidage.

Un même effet peut être obtenu si, conformément à la figure 11, la petite branche 82a du levier 82 est pourvue d'une surface 84 en dièdre rentrant pouvant pénétrer dans une rainure 85 de forme complémentaire prévue dans la surface périphérique du prolongement 75.

**Revendications**

1. Appareil distributeur nécessitant pour fonctionner l'emploi d'éléments consommables se présentant sous forme de bandes (34), chacune de ces bandes comprenant dans le sens longitudinal plusieurs zones d'utilisation successives (Z1 à Z9) destinées à être séparées de la bande dès après leur utilisation, ledit appareil comprenant:

   - un boîtier (20) définissant un couloir de circulation (29) pour lesdites bandes, ledit couloir présentant, compte tenu du sens d'introduction desdites bandes, une extrémité amont (AM) et une extrémité aval (AV),
   - un mécanisme d'avance (30) pour faire sortir ladite bande (34) du couloir de circulation (29) selon un mouvement pas-à-pas (F1) après son introduction dans celui-ci par un utilisateur, et
   - une unité coulissante (31) montée mobile dans ledit couloir (29), couplée fonctionnellement audit mécanisme d'avance (30) et destinée à être accouplée à l'extrémité aval (AV) de ladite bande (34) lors de son introduction par l'utilisateur, ledit appareil distributeur étant caractérisé en ce qu'il comporte également des moyens de commande (58, 74, 75) pour permettre le recul de ladite bande (34) sur une distance prédéterminée (w) inférieure à un pas d'avancement (clo) de cette bande après qu'elle ait exécutée un tel pas.

2. Appareil distributeur selon la revendication 1, caractérisé en ce qu'il comporte également des moyens de coupe (32) destiné à tronçonner ladite bande consommable (34) et en ce que lesdits moyens de commande (58, 74, 75) sont couplés fonctionnellement auxdits moyens de coupe (32) pour que ceux-ci ne puissent tronçonner ladite bande (34) qu'après que lesdits moyens de commande aient provoqué le recul de ladite bande (34).

3. Appareil distributeur selon l'une quelconque des revendications 1 et 2, caractérisé en ce que lesdits moyens de commande (58, 74, 75) sont agencés pour permettre également l'avancement de ladite bande (34) sur une distance (w) inférieure à un pas d'avancement de cette bande préalablement à l'exécution d'un pas d'avancement (clo).

4. Appareil distributeur selon l'une quelconque des revendications 1 à 3, caractérisé en ce qu'il comporte un couvercle mobile (22) entre une position d'ouverture et une position de fermeture et en ce que lesdits moyens de commande (58, 74, 75) sont couplés audit couvercle pour être actionnés lors du ou des mouvements d'ouverture respectivement. de fermeture dudit couvercle (22).

5. Appareil distributeur selon la revendication 4, caractérisé en ce que lesdits moyens de commande (58, 74, 75) comprennent un dispositif (76;

82) d'amplification du mouvement dudit couvercle (22).

6. Appareil distributeur selon l'une quelconque des revendications 1 à 5, caractérisé en ce qu'il comprend également :

- une unité coulissante (31) montée mobile dans ledit couloir (29), couplée fonctionnellement audit mécanisme d'avance (30) et destinée à être accouplée à l'extrémité aval (AV) de ladite bande (34) lors de son introduction par l'utilisateur, et
- des moyens élastiques (41) portées par ladite unité coulissante (31) et agencés pour assurer le couplage entre celle-ci et ladite bande (34), et en ce que ladite unité coulissante (31) comprend un curseur (35) monté coulissant dans ledit couloir de ce circulation (29) et muni d'un organe d'entraînement (72) destiné à coopérer avec lesdits moyens d'avance (30).

7. Appareil distributeur suivant la revendication 6, caractérisé en ce que ledit mécanisme d'avance (30) comprend des moyens d'encliquetage (54, 57, 62a à 67) destinés à coopérer avec ledit organe d'entraînement (72) pour assurer ledit couplage et permettre l'avance pas-à-pas dudit curseur (35), et en ce que lesdits moyens d'encliquetage (54, 57, 62a à 67) sont couplés à un bouton d'actionnement (23) monté mobile dans ledit boîtier (20) pour permettre la commande dudit mouvement pas-à-pas.

8. Appareil distributeur suivant la revendication 7, caractérisé en ce que lesdits moyens d'encliquetage comprennent une barre d'encliquetage (57) montée dans ledit boîtier parallèlement audit couloir de circulation (29) de manière à pouvoir exécuter un mouvement orbital (F5 à F8) dans son propre plan, ladite barre d'encliquetage présentant des moyens pour, au cours de chaque mouvement orbital correspondant à l'avance d'un pas dudit curseur (35), venir en prise avec ledit organe d'entraînement (72) de celui-ci.

9. Appareil distributeur selon la revendication 8, caractérisé en ce que ledit mouvement orbital (F5 à F8) présente un tracé rectangulaire.

10. Appareil distributeur selon l'une quelconque des revendications 8 et 9, caractérisé en ce que ladite barre d'encliquetage (57) comprend une denture à dents de scie (59) dont les dents (60) sont destinées à venir en prise avec ledit organe d'entraînement (72).

11. Appareil distributeur selon l'une quelconque des revendications 8 à 10, caractérisé en ce que ladite barre d'encliquetage (57) présente au moins une découpe (63a, 63b) dans laquelle est engagé un organe suiveur (67) fixe par rapport audit boîtier (20) pour définir avec ladite découpe (63a, 63b) ledit mouvement orbital (F5 à F8).

12. Appareil distributeur suivant l'une quelconque des revendications 8 à 11, caractérisé en ce qu'il est prévu des moyens élastiques de rappel (54) sollicitant ladite barre d'encliquetage (57) dans le sens opposé au sens d'introduction de ladite barre consommable (34) dans ledit couloir de circulation (29).

13. Appareil distributeur suivant l'une quelconque des revendications 1 à 12, caractérisé en ce que des crans d'indexation (44) sont prévus dans ledit couloir de circulation (29) au pas dudit mouvement pas-à-pas et en ce que ledit organe d'entraînement (72) est agencé pour venir en prise avec lesdits crans d'indexation (44) pour fixer chaque position dudit curseur (35).

FIG.:1

EP 0 740 959 A1

FIG.:2A

FIG.:2B

FIG.:2C

FIG.:3

FIG.:4

FIG.: 5

EP 0 740 959 A1

FIG.: 5A

FIG.:1A

FIG.:6

FIG.:6A

FIG.: 7

FIG.:8

FIG.:9A

FIG.:9B

FIG.: 10A

FIG.: 10B

FIG.: 11

**Office européen des brevets**

# RAPPORT DE RECHERCHE EUROPEENNE

Numero de la demande

EP 96 10 6413

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,Y<br>A | FR-A-2 710 411 (ASULAB SA) 31 Mars 1995<br>* le document en entier *<br>--- | 1<br>2,4 | B01L11/00<br>G01N27/28<br>B65D83/08 |
| Y<br><br>A | EP-A-0 416 507 (TOKYO ELECTRIC CO LTD) 13 Mars 1991<br>* colonne 4, ligne 17 - colonne 5, ligne 31 *<br>--- | 1<br><br>2,3 | |
| A | US-A-5 014 429 (MCNAMARA WILLIAM J) 14 Mai 1991<br>* colonne 6, ligne 46 - colonne 7, ligne 38 *<br>--- | 1-3 | |
| D,A | FR-A-2 710 412 (ASULAB SA) 31 Mars 1995<br>* le document en entier *<br>--- | 6,7 | |
| A | US-A-4 240 202 (GILBERT R) 23 Décembre 1980<br>* le document en entier *<br>----- | 6,7 | |

**DOMAINES TECHNIQUES RECHERCHES (Int.Cl.6)**

B65D
G03B
G01N
B01L

Le présent rapport a été établi pour toutes les revendications

| Lieu de la recherche | Date d'achèvement de la recherche | Examinateur |
|---|---|---|
| LA HAYE | 31 Juillet 1996 | Bindon, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

............................................................................................

& : membre de la même famille, document correspondant

EPO FORM 1503 03.82 (P04C02)